# EUROPÄISCHE PATENTANMELDUNG

(11) **EP 1 454 601 A2**
(43) Veröffentlichungstag der Anmeldung: **08.09.2004**
(21) Anmeldenummer: 04004790.4
(22) Anmeldetag: 02.03.2004
(51) Int. Cl.: A61F 2/20

(54) **Anordnung zur externen Tracheostomastabilisierung**

(30) Priorität: 06.03.2003 DE 20303669 U
(71) Anmelder: bess medizintechnik gmbh, 14167 Berlin (DE)
(72) Erfinder: Schäfer, Peter, Dr.Med., 54317 Güsterath (DE); Schneider, Mathias, 66482 Zweibrücken (DE)
(74) Vertreter: Griepenstroh, Jörg

(57) **Zusammenfassung**

Anordnung zur externen Tracheostomastabilisierung, mit einem äußerlich an einem Tracheostoma zumindest mittelbar anliegenden und zumindest teilweise in ein Tracheostoma hineinragenden Tracheostomastabilisierungsring (TSR) (1,21), welcher an seinem Außenumfang (6,7) wenigstens zwei Kupplungsmittel (8,9) besitzt, an denen jeweils ein Ende eines um den Hals des Trägers führbaren elastischen Tragebandes anbringbar ist.

## Beschreibung

Die Erfindung betrifft eine Anordnung zur externen Tracheostomastabilisierung gemäß den Merkmalen des Schutzanspruchs 1.

Die Stimmenrehabilitation nach der Laryngektomie wurde bereits von Billroth 1873 im Rahmen der ersten Laryngektomie thematisiert. Damals wurde eine Sprechkanüle nach Gussenbauer konstruiert, mit deren Hilfe der Patient sprechen konnte. Aufgrund von Aspirationen musste diese Technik verlassen werden, so dass als einzige Methode der Stimmbildung die Oesophagus-Ersatzstimme zur Verfügung stand. In den 60er und 70er Jahren des letzten Jahrhunderts konnten sich andere operative Techniken, wie z.B. die Staffieri-Plastik, langfristig nicht etablieren. Erst die 1980 von Blom und Singer entwickelte Stimmprothese brachte eine Wende. Fingerfreies Sprechen war 1982 mit einem Blom-Singer-Tracheostomaventil ebenfalls bereits möglich. In den nächsten Jahren kamen zahlreiche Weiterentwicklungen hinzu, wie z.B. das in Deutschland konstruierte ESKA-Herrmann-Ventil sowie die sogenannte Provox-Prothese. Neue Tracheostomaventile, wie das Window- oder das FreeHands-Ventil sollen den Anteil der Patienten, die fingerfrei sprechen, erhöhen.

Zur Fixierung des Ventils am Tracheostoma finden HME-(Heat and Moisture Exchanger)-Pflaster, Barton-Mayo-Button und spezielle Kanülen bzw. Tracheostomaplatzhalter Verwendung. In besonderen Fällen kann ein Tracheostomaabdruck angefertigt werden. Aufwendige operative Rehabilitationsmaßnahmen wie die Laryngoplastik finden in einzelnen Kliniken Anwendung, stellen jedoch aufgrund des aufwendigen Verfahrens keine ubiquitär anwendbare Operationstechnik dar. Ein Trachealkamin wird nur vereinzelt operativ angelegt. Das direkte Vernähen des Trachealstumpfs mit der Haut ist weit verbreitet.

Mit den standardmäßig vorhandenen Klebern und Barton-Mayo-Buttons kann in ca. 10 % der Fälle eine optimale Abdichtung des Tracheostomas herbeigeführt werden. Ein individueller Tracheostomaabdruck sollte nur als letzte mögliche Technik herangezogen werden, da dieses Verfahren sehr aufwendig ist und erfahrungsgemäß nicht immer zum fingerfreien Sprechen führt.

Hiervon ausgehend liegt der Erfindung die Aufgabe zugrunde, eine Anordnung zur Tracheostomastabilisierung aufzuzeigen, welche eine verbesserte Tracheostomaabdichtung ermöglicht und somit auch bei hohen Drücken, wie sie beim fingerfreien Sprechen auftreten, zu einer zuverlässigen Stabilisierung eines Tracheostomas führt.

Diese Aufgabe ist bei einer Anordnung mit den Merkmalen des Schutzanspruchs 1 gelöst.

Die erfindungsgemäße Anordnung zur externen Tracheostomastabilisierung umfasst einen äußerlich an einem Tracheostoma zumindest mittelbar anliegenden und/oder zumindest teilweise in ein Tracheostoma hinein ragenden Tracheostomastabilisierungsring (nachfolgend TSR genannt). Dieser TSR ist über ein Trageband, das um den Hals eines Trägers des TSR gelegt ist, fixiert, wobei das Trageband mit jeweils einem Ende an Kupplungsmitteln am Außenumfang des TSR befestigt ist. Das Trageband ist elastisch und in der Länge verstellbar, so dass die erfindungsgemäße Anordnung individuell auf den Träger des TSR abstimmbar ist.

Der TSR besteht vorzugsweise aus Kunststoff, insbesondere aus Silikon. Der TSR ist vorzugsweise flexibel und passt sich in gewissen Grenzen an die Auflagefläche rund um das Tracheostoma an. Der TSR kann für sich allein oder zusammen mit am Tracheostoma aufgeklebten Tracheostomaventilen bzw. aufgeklebten Halterungen für derartige Ventile zum Einsatz kommen, wobei der TSR einen hinreichend großen zentralen Durchlass aufweist, der das Tracheostomaventil umgibt und somit die Klebeflächen des aufgeklebten Tracheostomaventils über das elastische Trageband lagefixiert. Über das elastische Trageband wird ein zusätzlicher Druck auf die Klebeflächen ausgeübt, so dass eine verbesserte Tracheostomaabdichtung gegeben ist.

Insbesondere wenn es sich bei dem Tracheostomaventil um ein automatisches Sprechventil handelt, ist eine zuverlässige umfangsseitige Abdichtung des Sprechventils gegenüber dem Tracheostoma erforderlich, damit ein Mindestanblasdruck aufrecht erhalten werden kann. Studien haben gezeigt, dass der Trachealdruck während des fingerfreien Sprechens bei ca. 3 kP liegt. Patienten, bei denen die Tracheostomaabdichtung nicht möglich ist, zeigen einen doppelt so hohen Trachealdruck mit Maximalwerten von über 10 kP. Bei derart hohen Drücken ist es nicht ausgeschlossen, dass sich aufgeklebte Tracheostomaventile zumindest bereichsweise lösen, was bereits zu einem erheblichen Druckverlust und damit zu einer Behinderung des fingerfreien Sprechens führen kann. Mit der erfindungsgemäßen Anordnung einer externen Tracheostomastabilisierung ist es gemäß Studien aufgrund der verbesserten Dichtwirkung ungefähr jedem fünften Patient möglich, das fingerfreie Sprechen zu erlernen.

Dies wird insbesondere dadurch erreicht, dass der TSR zumindest teilweise in ein Tracheostoma hinein ragt. Unter Hineinragen ist im Sinne der Erfindung nicht zu verstehen, dass sich der TSR selbst in die Trachea erstreckt, sondern lediglich in das Tracheostoma. Der TSR besitzt hierfür einen mittleren Bereich, der vorzugsweise trichterförmig ausgebildet ist und in das Tracheostoma einragt. Die trichterförmige Ausprägung dient insbesondere dazu, ein das Tracheostoma umgebende Pflaster im kritischen Übergangsbereich zum Tracheostoma selbst zusätzlich anzudrücken, um dadurch einen Gegendruck zur Ausatmung auszuüben. Ein weiterer Vorteil ist, dass der in das Tracheostoma hinein ragende mittlere Bereich des TSR gegenüber Randbereichen des TSR, die äußerlich auf dem Hals des Trägers aufliegen, zurückversetzt ist, so dass an dem mittleren Bereich befestigte HME-Einheiten oder Tracheostomaventile weniger weit über das Tracheostoma vorstehen und somit unauffälliger zu tragen sind. Insbesondere können auf diese Weise unschöne Konstruktionen vermieden werden, die dazu führen, dass Betroffene eine deutlich sichtbare Prothese tragen und so auf den ersten Blick als Behinderte stigmatisiert werden. Die erfindungsgemäße Anordnung zur externen Tracheostomastabilisierung ermöglicht ein unauffälliges Tragen eines HME-Pflasters bzw. eines Tracheostomaventils, insbesondere wenn zusätzlich ein Schal oder ein anderes den Halsbereich bedeckendes Kleidungsstück getragen wird.

Gemäß den Merkmalen des Schutzanspruchs 2 sind die Kupplungsmittel von dem elastischen Trageband durchsetzte Laschen. Laschen oder Ösen sind aufgrund der unterschiedlichen Werkstoffe des Tragebands und des TSR die konstruktiv einfachste Lösung einer zuverlässigen Verbindung dieser beiden Komponenten. Selbstverständlich ist es auch möglich, andersartig gestaltete Kupplungsmittel vorzusehen, beispielsweise in Form von ersten Kupplungsteilen, an denen zweite Kupplungsteile formschlüssig oder kraftschlüssig festglegbar sind, die wiederum mit dem Trageband verbunden sind. Selbstverständlich ist es auch möglich, das Trageband unlösbar an dem TSR zu fixieren, wobei das Kupplungsmittel eine stoffschlüssige, formschlüssige oder kraftschlüssige Verbindung zwischen den Enden des Tragebands und dem TSR ist.

Nach den Merkmalen des Schutzanspruchs 3 sind die Kupplungsmittel diametral angeordnet. Es hat sich gezeigt, dass die diametrale Anordnung aufgrund der von dem Trageband gleichmäßig aufgebrachten Zugkräfte für eine in den meisten Fällen optimale Positionierung und externe Stabilisierung des Tracheostomas führt. Je nach Position des Tracheostomas bzw. des TSR verläuft das Trageband mehr oder weniger V-förmig, wobei der TSR am tiefsten Punkt angeordet ist, so dass sich eine nach oben wirkende Kraftkomponente bzw. Zugkraft ergibt. Diese nach oben wirkende Zugkraft kann dadurch aufgefangen werden, dass am Außenumfang des TSR eine im gleichmäßigen Abstand zu den Kupplungsmitteln angeordnete radial nach außen weisende Zunge vorgesehen ist (Schutzanspruch 4), wobei diese Zunge so orientiert ist, dass sie entgegen der nach oben ziehenden Kraftkomponente des Tragebands angeordnet ist, das heißt nach unten in Richtung der Trachea weist und hier beispielsweise klebetechnisch fixiert werden kann. Dies kann durch handelsübliches Heftpflaster erfolgen, das über die Zunge gelegt wird. Die Zunge kann aber selbst auch als Lasche oder Öse ausgeführt sein, so dass gegebenenfalls ein weiteres elastisches Trageband durch diese Lasche oder Öse geführt werden kann, das in geeigneter Weise Zug nach unten, das heißt in Richtung der Trachea ausübt.

Nach den Merkmalen des Schutzanspruchs 5 sind die Zunge und die Kupplungsmittel jeweils um 90° versetzt zueinander angeordnet.

Der TSR kann im Rahmen der beanspruchten Anordnung ausschließlich durch das Trageband lageorientiert sein oder auch zusätzlich mit einer Haftbeschichtung versehen sein, wie dies im Rahmen des Schutzanspruchs 6 vorgeschlagen wird. Die Haftbeschichtung kann zumindest teilweise oder auch vollflächig auf der Auflagerfläche vorgesehen sein. Die Ausführungsform ohne Haftbeschichtung eignet sich insbesondere für Personen, die allergisch auf bestimmte Haftbeschichtungen reagieren.

Während durch eine Haftbeschichtung bei einem relativ ebenen Tracheostoma eine sehr gute Tracheostomaabdichtung erzielbar ist, kann bei einem unregelmäßigen Tracheostoma ein flexibles Auflagerkissen an der dem Tracheostoma zugewandten Seite des TSR vorgesehen sein (Schutzaspruch 7). Ein solches Auflagerkissen kann gemäß den Merkmalen des Schutzanspruchs 8 mit einem Fluid befüllbar sein. Das Auflagerkissen kann bereits als vorkonfigurierte Einheit vollständig mit einem Fluid befüllt sein, beispielsweise mit einem Gas, einer Flüssigkeit oder auch mit einem Gel. Es ist auch denkbar, dass das Auflagerkissen nachträglich über ein geeignetes Ventil mit beispielsweise Luft oder Wasser befüllt werden kann.

Das Auflagerkissen kann gemäß den Merkmalen des Schutzanspruchs 9 allerdings auch aus einem porösen Werkstoff bestehen. Poröse Werkstoffe, die bis zu einem gewissen Grad kompressibel und selbstexpandierend sind, wie beispielsweise Schaumstoffe, z.B. PVA, sind ebenfalls geeignet, den TSR insbesondere bei unregelmäßigen und/oder tiefen Tracheostoma flächig zur Anlage zu bringen.

Der TSR kann herkömmliche aufgeklebte HME-Pflaster bzw. Tracheostomaventile dadurch ersetzen, dass auch an dem erfindungsgemäßen TSR ein Tracheostomaventil anbringbar ist. Der TSR dient somit gewissermaßen als Adapter zur Aufnahme unterschiedlicher medizinischer Funktionskomponenten, wie beispielsweise eines Schlauchs und/oder trichterförmigen Stutzens, wie er Gegenstand des Schutzanspruchs 10 ist.

Auch kann an dem TSR eine Trachealkanüle lösbar oder unlösbar befestigt sein (Schutzanspruch 11).

Als vorteilhaft wird es ferner angesehen, wenn der TSR Kupplungsmittel zur lösbaren Fixierung einer Epithese aufweist (Schutzanspruch 12).

Insbesondere weist der TSR gemäß den Merkmalen des Schutzanspruchs 13 eine zentrale Aufnahme zur lösbaren Fixierung eines Tracheostomaverschlusses oder eines Tracheostomafilters auf. Die zentrale Aufnahme dient als standardisierte Halterung für unterschiedliches Zubehör, wie beispielsweise für HME-Kassetten, Sprechventile unterschiedlicher Ausführungsformen oder auch für diverses Zubehör, insbesondere zur Ermöglichung eines leichten manuellen Tracheostomaverschlusses bei unregelmäßigen und/oder tiefen Tracheostoma.

Die Erfindung wird nachfolgend anhand eines in den Zeichnungen dargestellten Ausführungsbeispiels näher erläutert. Es zeigen:
- Figuren 1 und 2: einen Tracheostomastabilisierungsring in zwei unterschiedlichen Seitenansichten;
- Figur 3: den Tracheostomastabilisierungsring der Figuren 1 und 2 von oben;
- Figur 4: eine perspektivische Darstellung des Tracheostomastabilisierungsrings der Figuren 1 bis 3;
- Figur 5: eine weitere Ausführungsform eines Tracheostomastabilisierungsrings in der Draufsicht;
- Figuren 6 und 7: den Tracheostomastabilisierungsring der Figur 5 in zwei Seitenansichten;
- Figur 8: einen Schnitt durch den Tracheostomastabilisierungsring der Figur 5 in Blickrichtung des Pfeils VIII-VIII;
- Figur 9: eine perspektivische Darstellung des Tracheostomastabilisierungsrings der Figur 5;
- Figur 10: eine schematische Darstellung der Anordnung eines Tracheostomastabilisierungsrings an einem Menschen und
- Figur 11: einen Schnitt durch einen Trachestomatisierungsring mit einer HME-Einheit.

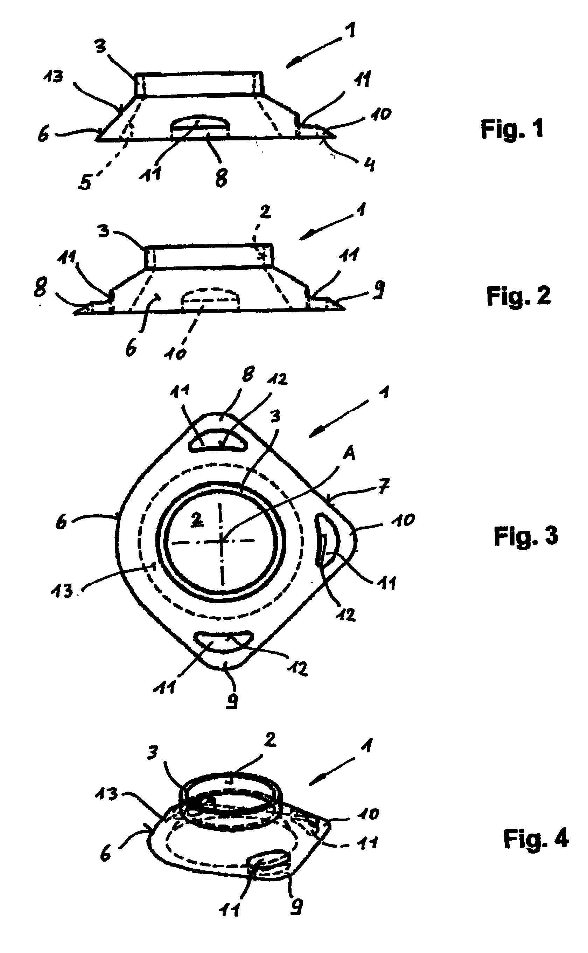

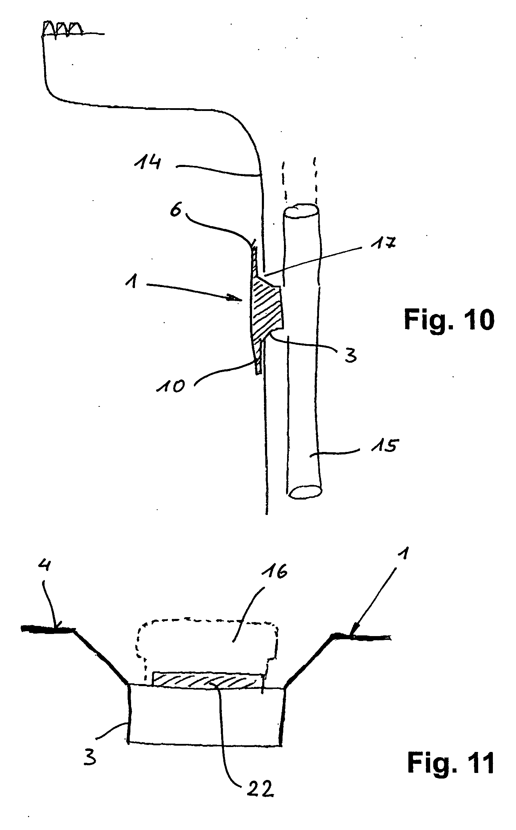

Der in den Figuren 1 bis 4 dargestellte Tracheostomastabilisierungsring (TSR) 1 ist Bestandteil einer Anordnung zur externen Tracheostomastabilisierung, die neben dem TSR 1 noch ein nicht näher dargestelltes Trageband umfasst. Der TSR 1 ist ringförmig konfiguriert mit einer zentralen kreisrunden Öffnung 2. Die Öffnung 2 liegt innerhalb eines sich in axialer Richtung der Öffnung 2 erstreckenden Axialkragens 3, der zur Aufnahme von Zubehörkomponenten, insbesondere eines Tracheostomafilters oder eines Tracheostomaverschlusses, dient. Der Axialkragen 3 ist auf einer der Außenfläche 4 abgewandten Seite des TSR 1 und somit zu dem Tracheostoma hin weisend angeordnet. Der TSR 1 liegt mit der der Außenfläche 4 abgewandten Auflagerfläche 13 auf einem Tracheostoma 17 auf (Figur 10).

Figur 10 zeigt eine schematische Darstellung der Anordnung des TSR 1 am Hals 14 eines Trägers, wobei deutlich wird, dass der Axialkragen 3 der Trachea 15 zugewandt ist, ohne selbst in diese zu ragen. Der TSR 1 bzw. der Axialkragen 3 ist lediglich innerhalb des Tracheostomas 17 angeordnet.

Figur 11 verdeutlicht die Aufnahme einer HME-Einheit 16 innerhalb des TSR 1, wobei die HME-Einheit 16 nicht oder nicht wesentlich über die Außenfläche 4 des TSR 1 vorsteht.

Innerhalb der Öffnung 2 des Axialkragens 3 ist in diesem Ausführungsbeispiel eine Aufnahme 22 angeordnet, an welcher die HME-Einheit 16 lösbar befestigt ist. Die Aufnahme 22 dient aber grundsätzlich zur lösbaren Fixierung beliebiger passender Zubehörkomponenten, insbesondere zur Fixierung eines Tracheostomafilters oder Tracheostomaverschlusses, die sich jeweils von dem Axialkragen 3 weg in Richtung zur Außenfläche 4 erstrecken.

Zwischen der Außenfläche 4 und dem Axialkragen 3 ist ein sich konisch in Richtung des Axialkragens 3 verjüngender Übergangsabschnitt 5 vorgesehen (Figur 1), wobei durch die konische Ausbildung die Öffnung 2 im Bereich der Außenfläche 4 einen größeren Durchmesser aufweist als im Bereich des Axialkragens 3. Der Durchmesser der Öffnung 2 im Bereich der Außenfläche 4 ist mit unterbrochener Linie eingezeichnet (Figur 3). Mit zunehmender Vergrößerung der Öffnung 2 nimmt auch die Wanddicke ausgehend vom Axialkragen 3 in Richtung zur Außenfläche 4 hin zu.

Aus Figur 3 wird deutlich, dass der TSR 1 einen gerundeten Außenumfangsbereich 6 aufweist, der konzentrisch zur Öffnung 2 und zum Axialkragen 3 ausgebildet ist. Der gerundete Außenumfangsbereich 6 geht tangential über in einen zweiten eckigen Außenumfangsbereich 7 mit drei, jeweils um 90° bezogen auf eine Axialachse A der Öffnung 2, versetzten Eckbereichen, wobei die diametral angeordneten Eckbereiche Kupplungsmittel 8, 9 in Form von Laschen sind, die von dem nicht näher dargestellten elastischen Trageband durchsetzt sind. Am Hals 14 eines Trägers weist der gerundete Außenumfangsbereich 6 nach oben, wobei die lateralen Kupplungsmittel 8, 9 jeweils horizonal orientiert sind. Der dritte Eckbereich ist dann eine nach unten weisende Zunge 10, die entweder über ein weiteres Trageband fixiert werden kann oder durch Überkleben mit einem Heftpflaster am Hals 14 des Trägers fixierbar ist. Die Kupplungsmittel 8, 9 und die Zunge 10 sind identisch ausgebildet und weisen jeweils eine sichelförmige Aussparung 11 auf, wobei die konkave Seite 12 der Aussparungen 11 jeweils der Mittellängsachse A der Öffnung 2 zugewandt ist.

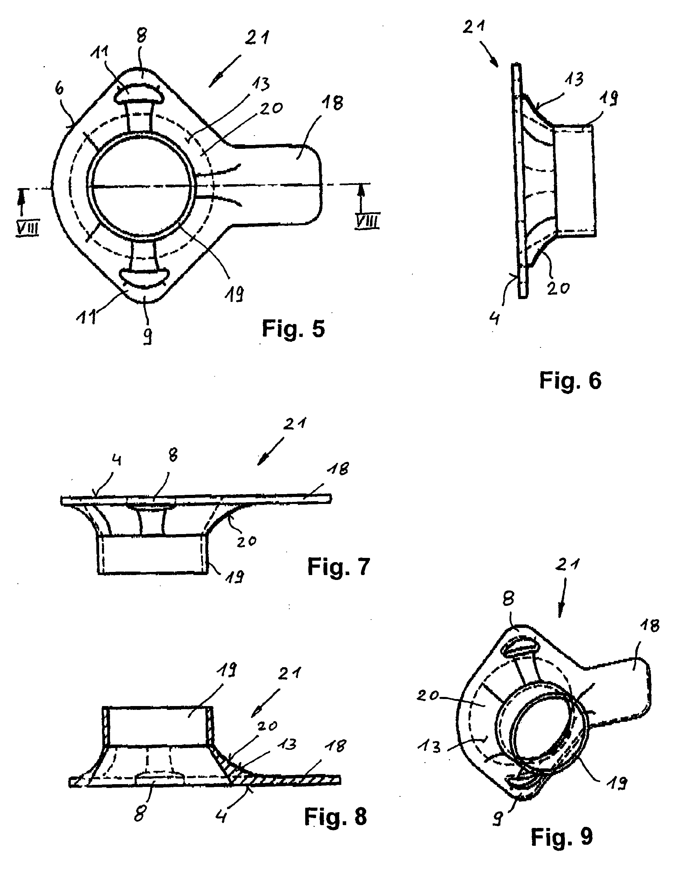

Die Ausführungsform der Figuren 5 bis 9 unterscheidet sich von derjenigen der Figuren 1 bis 4 durch eine wesentlich vergrößert gestaltete Zunge 18, die in diesem Ausführungsbeispiel rechteckig konfiguriert ist und beispielsweise eine in Richtung der Kupplungsmittel 8, 9 gemessene konstante Breite von beispielsweise 20 mm besitzen kann. Ferner ist der Axialkragen 19 bei dieser Ausführungsform deutlich höher bzw. länger bemessen als bei der Ausführungsform der Figuren 1 bis 4. Ein weiterer Unterschied ist, dass der Übergangsabschnitt 20 zwischen dem Axialkragen 19 und der Außenfläche 4 außenseitig gerundet ausgeführt ist, so dass sich ein fließender Übergang zwischen dem Axialkragen 19 in Richtung zum Außenumfangsbereich 6 ergibt. Aufgrund der in diesem Ausführungsbeispiel verlängerten Zunge 18 ist der Übergang zwischen dem Axialkragen 19 und der Zunge 18 gerundet, wobei die Zunge 18 selbst konstante Dicke besitzt.

### Bezugszeichenaufstellung

- 1 -: TSR
- 2 -: Öffnung in 1
- 3 -: Axialkragen
- 4 -: Außenfläche
- 5 -: Übergangsabschnitt zw. 3 u. 4
- 6 -: Außenumfangsbereich
- 7 -: Außenumfangsbereich
- 8 -: Kupplungsmittel
- 9 -: Kupplungsmittel
- 10 -: Zunge
- 11 -: Aussparung in 8, 9, 10
- 12 -: Seite v. 11
- 13 -: Auflagerfläche
- 14 -: Hals
- 15 -: Trachea
- 16 -: HME-Einheit
- 17 -: Tracheostoma
- 18-: Zunge
- 19 -: Axialkragen
- 20-: Übergangsabschnitt
- 21 -: TSR
- 22-: Aufnahme

- A -: Achse

## Patentansprüche

1. Anordnung zur externen Tracheostomastabilisierung, umfassend einen äußerlich an einem Tracheostoma zumindest mittelbar anliegenden und zumindest teilweise in ein Tracheostoma (17) hineinragenden Tracheostomastabilisierungsring (TSR) (1, 21), welcher an seinem Außenumfang (6, 7) wenigstens zwei Kupplungsmittel (8, 9) besitzt, an denen jeweils ein Ende eines um den Hals (14) eines Trägers führbaren elastischen Tragebandes anbringbar ist.

2. Anordnung nach Anspruch 1, **dadurch gekennzeichnet, dass** die Kupplungsmittel (8, 9) von dem elastischen Trageband durchsetzte Laschen sind.

3. Anordnung nach Anspruch 1 oder 2, **dadurch gekennzeichnet, dass** die Kupplungsmittel (8, 9) diametral angeordnet sind.

4. Anordnung nach einem der Ansprüche 1 bis 3, **dadurch gekennzeichnet, dass** am Außenumfang des TSR (1) eine im gleichmäßigen Abstand zu den Kupplungsmitteln (8, 9) angeordnete radial nach außen weisende Zunge (10, 18) vorgesehen ist.

5. Anordnung nach Anspruch 4, **dadurch gekennzeichnet, dass** die Zunge (10, 18) und die Kupplungsmittel (8, 9) jeweils um 90° versetzt zueinander angeordnet sind.

6. Anordnung nach einem der Ansprüche 1 bis 4, **dadurch gekennzeichnet, dass** eine einem Tracheostoma (17) zugewandte Auflagerfläche (13) des TSR (1) mit einer Haftbeschichtung versehen ist.

7. Anordnung nach einem der Ansprüche 1 bis 5, **dadurch gekennzeichnet, dass** der TSR (1) an seiner einem Tracheostoma (17) zugewandten Seite mit einem flexiblen Auflagerkissen versehen ist.

8. Anordnung nach Anspruch 7, **dadurch gekennzeichnet, dass** das Auflagerkissen mit einem Fluid befüllt oder befüllbar ist.

9. Anordnung nach Anspruch 7, **dadurch gekennzeichnet, dass** das Auflagerkissen aus einem porösen Werkstoff besteht.

10. Anordnung nach einem der Ansprüche 1 bis 9, **dadurch gekennzeichnet, dass** an dem TSR (1, 21) ein trichterförmiger Stutzen und/oder ein in Richtung eines Tracheostomas (17) weisender Schlauch vorgesehen ist.

11. Anordnung nach einem der Ansprüche 1 bis 10, **dadurch gekennzeichnet, dass** an dem TSR (1, 21) eine Trachealkanüle oder ein Tracheostomaplatzhalter lösbar oder unlösbar befestigt ist.

12. Anordnung nach einem der Ansprüche 1 bis 11, **dadurch gekennzeichnet, dass** der TSR (1, 21) Kupplungsmittel zur lösbaren Fixierung einer Epithese aufweist.

13. Anordnung nach einem der Ansprüche 1 bis 12, **dadurch gekennzeichnet, dass** der TSR (1, 21) eine zentrale Aufnahme (3, 19, 22) zur lösbaren Fixierung eines Tracheostomaverschlusses oder eines Tracheostomafilters besitzt.
